# EUROPEAN PATENT SPECIFICATION

(11) **EP 2 273 900 B1**
(45) Date of publication and mention of the grant of the patent: **10.04.2019**
(21) Application number: 09747351.6
(22) Date of filing: 12.05.2009
(51) Int. Cl.: B32B 3/10, B32B 7/12, B32B 27/08, B32B 27/10, B32B 27/04, B32B 27/14, B32B 7/04, B32B 7/06, B32B 27/16, B32B 27/18, B32B 27/20, B32B 27/28, B32B 27/30, B32B 27/32, B32B 27/36, B32B 27/40, G09F 3/00

(54) **PRINTABLE FORM HAVING DURABLE RESISTANT WRISTBAND AND LABELS**
BEDRUCKBARE FORM MIT LANGLEBIGEM RESISTENTEM ARMBAND UND ETIKETTEN
FORMULAIRE IMPRIMABLE PRÉSENTANT DES ÉTIQUETTES ET UN BRACELET RÉSISTANTS ET DURABLES

(30) Priority: 13.05.2008 US 52683
(43) Date of publication of application: 19.01.2011
(73) Proprietor: PRECISION DYNAMICS CORPORATION, Valencia, CA 91355 (US)
(72) Inventor: TAGHAVI, Shane, San Fernando, CA 91340 (US); CHILDERS, Winthrop, D., San Diego, CA 92127 (US)
(74) Representative: RGTH
(86) International application number: PCT/US2009/043610
(87) International publication number: WO 2009/140267

(56) References cited:
- WO-A1-99/25565
- US-A- 5 653 472
- US-A1- 2004 164 544
- US-A1- 2006 236 578
- US-A1- 2007 120 358
- US-B2- 7 000 951
- US-B2- 7 017 293

## Description

### BACKGROUND OF THE INVENTION

Form based wristbands, labels and/or tags for identification have been known in the prior art. Such identification forms have heretofore been made using standard paper label media as the top layer that receives identification information. The ability of these standard paper label media to resist fluids has been found to be inadequate for many applications. For example, in hospitals or settings involving exposure to fluids. Such inadequacy has previously been addressed by providing a lamination layer in addition to the printable media layer wherein the lamination layer is foldable over the media layer to protect the same from fluids. The addition of this lamination layer creates manufacturing and design issues such as increased cost, increased complexity, and increased user difficulty in properly aligning or sealing the lamination layer of the printable media layer. The WO 99/235565 discloses a laminated article (10) comprising a sheet having a wristband part with a die cut strip (12) and a part having die cut adhesive labels. The strip and labels are printed with correlated identification indicia. A release composition coats the underside of the wristband part with the exception of the strip. A transparent film coated with a pressure sensitive adhesive is releasably adhered to the underside of the wristband part and firmly adhered to the uncoated strip. The film includes two contiguous die cut rectangular areas, each slightly larger than the strip. To form a wristband, the rectangular areas are peeled from the sheet, and the rectangular area contiguous to the area bonded to the strip is folded on a longitudinally bisecting line separating the rectangular areas and adhered to the printed side of the strip. The labels can be affixed to medicine containers for a patient identified by the wristband.

Accordingly, there is a need for a durable wristband that along with an array of labels and/or tags can be printed on a standard printer without the need for a foldable lamination layer. In addition to being durable, the media layer should be solvent resistant and involve low cost, ease of manufacture, and ease of use. The present invention fulfills these needs and provides other related advantages.

### SUMMARY OF THE INVENTION

The present invention is directed to a printable identification medium. The identification medium preferably comprises at least one of a wristband, a label, a tag, or a sheet including a combination of a wristband, a label or a tag. In any of these forms the printable identification medium is configured for passing through a standard printer, i.e., laser printer, inkjet printer, thermal printer, electro-photography printer, or standard office printer.

The medium includes a polymeric substrate preferably made from polyethylene terephthalate (PET). A printable media layer is disposed adjacent to the polymeric substrate. The printable media layer preferably includes a core layer, an image receiving layer on a first side of the core layer, and an adhesive layer on an opposite second side of the core layer for bonding the printable media layer to the polymeric substrate. A release layer is preferably disposed between a portion of the adhesive layer and the polymeric substrate as discussed below.

The media layer preferably includes a wristband region, a label region, a tag region, or a combination thereof. Die cuts through both the printable media layer and polymeric substrate define a wristband within the wristband region. Die cuts through the printable media layer and polymeric substrate define a tag within the tag region. Die cuts through only the printable media layer define a label in the label region. An interior die cut within the defined wristband defines an attachment portion, wherein the interior die cut is only through the substrate. An interior die cut within the defined tag defines a tag attachment portion, wherein the interior die cut is only through the substrate.

In the label region, the core layer preferably comprises cellulose paper or synthetic paper. In the wristband and/or tag regions, the core layer preferably comprises a polymer. The image receiving layer preferably comprises urethane, polyethylene, polyethylene terephthalate (PET), vinyl, polyolefin, low-density polyethylene (LDPE), or high-density polyethylene (HDPE), each having a filler.

Other features and advantages of the present invention will become apparent from the following more detailed description, taken in conjunction with the accompanying drawings, which illustrate, by way of example, the principles of the invention.

### BRIEF DESCRIPTION OF THE DRAWINGS

The accompanying drawings illustrate the invention. In such drawings:
FIGURE 1 illustrates a layout of a printable form including a wristband portion and a plurality of label groups;
FIGURE 2 illustrates a cross-section of the printable form along line 2-2 of FIG. 1;
FIGURE 3 illustrates a partial cross-section of a liner layer of the present invention;
FIGURE 4 illustrates a partial cross-section of an alternate embodiment of a liner layer of the present invention;
FIGURE 5 illustrates a partial cross-section of a media layer of the present invention;
FIGURE 6 illustrates a partial cross-section of a media layer of an alternate embodiment of the present invention;
FIGURE 7 illustrates a partial cross-section of another alternate embodiment of a media layer of the present invention;
FIGURE 8 illustrates a configuration of a wristband of the present invention;
FIGURE 9 illustrates a cross-section of the wristband along line 9-9 of FIG. 8;
FIGURE 10 illustrates a cross-section of the wristband along line 10-10 of FIG. 8;
FIGURE 11 illustrates a cross-section of the wristband along line 10-10 of FIG. 8 after removal of the closure portion;
FIGURE 12 illustrates an alternate embodiment configuration of a wristband of the present invention;
FIGURE 13 illustrates a cross-section of the wristband of along line 13-13 of FIG. 12;
FIGURE 14 illustrates a cross-section of the wristband along line 14-14 of FIG. 12;

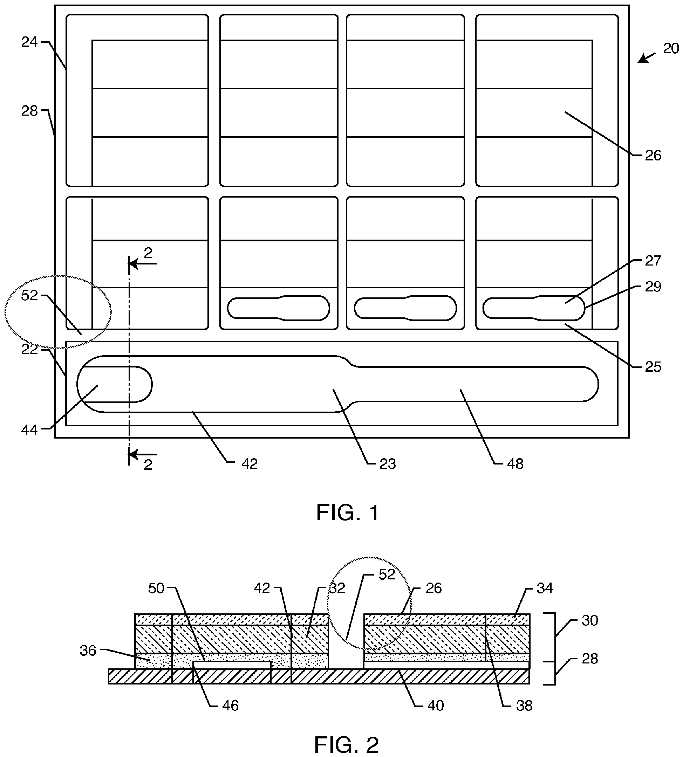

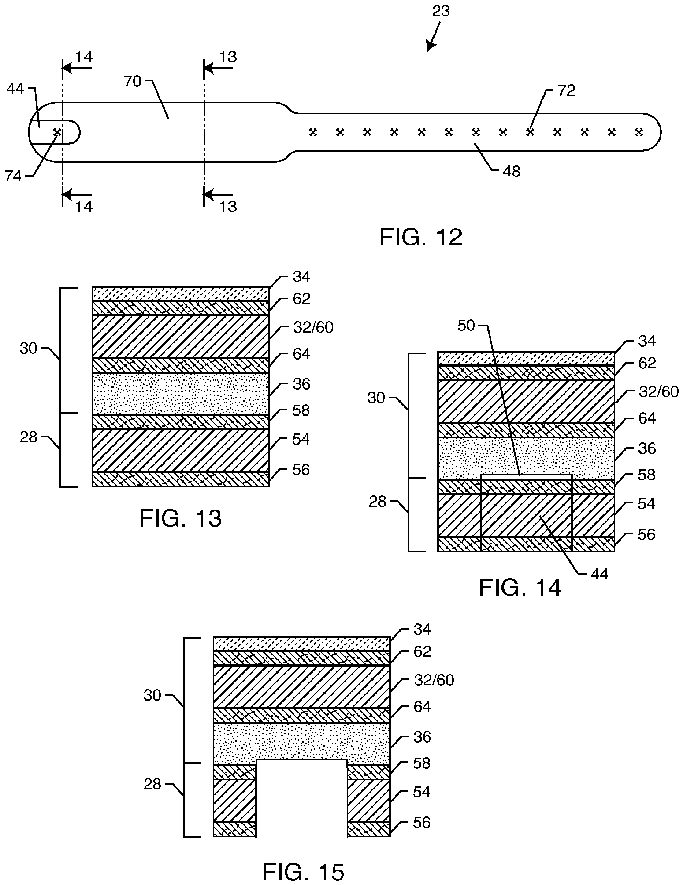

FIGURE 1 5 illustrates a cross-section of the wristband along line 14-14 of FIG. 12 after removal of the closure portion; and

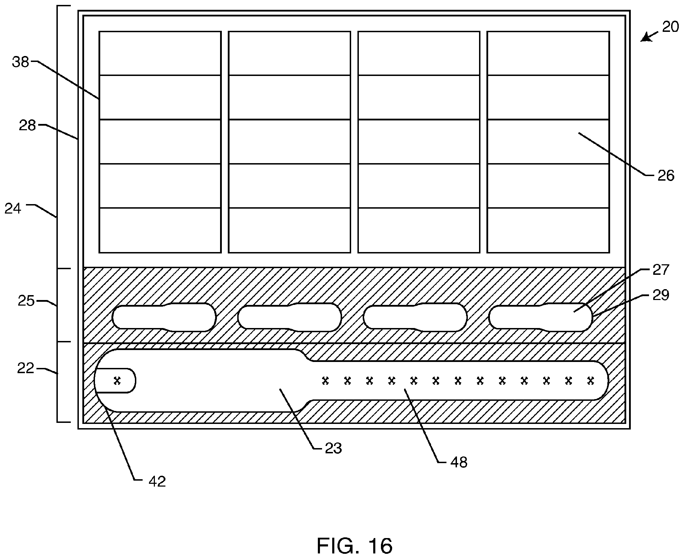

FIGURE 16 illustrates a layout of an alternate embodiment of the printable form including a wristband region, a tag region and a label region.

### DETAILED DESCRIPTION OF THE PREFERRED EMBODIMENTS

As shown in the exemplary drawings, for purposes of illustration, the present invention is concerned with form-based identification wristbands, labels and/or tags generally referred to by reference numeral 20. Different embodiments of the inventive form-based identification wristbands, labels and/or tags 20 will be described below. Throughout the description of each embodiment, the same reference numerals will be used to refer to similar components. In some cases where an inventive form 20 is depicted in cross-section, the thickness of the layers will be exaggerated for clarity. However, a person having ordinary skill in the art will understand that the depicted layers are much thinner than illustrated.

FIGURE 1 illustrates the layout of a first preferred embodiment of a form 20 of the present invention having an array of labels and a wristband. The form 20 includes a wristband portion or region 22 having a wristband 23 and a plurality of label groups or regions 24, each label group 24 including multiple labels 26. Some of the label groups 24 may include colored tags 27 as illustrated. The colored tags 27 may also be included in the wristband portion 22 or omitted entirely. In an alternate embodiment described below (FIG. 16), a separate tag region 25 may be included. The form 20 is configured to pass through a standard printer.

FIGURE 2 is a cross-section of a portion of the form 20 taken along line 2-2 of FIG. 1. This cross-section illustrates the various layers of the form 20. As illustrated, the form 20 consists primarily of a liner layer or polymeric substrate 28 and a printable media layer 30. The liner layer 28 is preferably made from a synthetic polymer material. The media layer 30 includes a core layer 32, an image receiving layer 34 on an upper surface thereof and an adhesive layer 36 on a lower surface thereof. It is at this adhesive layer 36 that the media layer 30 is joined to the liner layer 28.

In a label group or region 24, a series of discontinuous or continuous die cuts 38 through only the media layer 30 define each of the labels 26. A release layer 40 underlies the adhesive layer 36 under each of the labels 26. The release layer 40 allows each label 26 including the underlying portion of the adhesive layer 36 to be removed from the liner layer 28. The release layer 40 is preferably silicone but may be any other material commonly used for a release layer.

In a tag region 25, a series of continuous or discontinuous die cuts 29 through both the media layer 30 and the liner layer 28 define an outline of the tags 27. The cuts 29 are configured to enable the tag 27 to remain reliably attached to the form 20 during printing and handling but to be easily removed after printing in a manner such that media layer 30 and liner layer 28 remain permanently bonded together over most of the tag 27. A release layer 50 underlies a portion of the adhesive layer 36 on each tag 27 such that a portion of the underlying liner layer 28 is removable to expose the adhesive layer 36. In this way the tag 27 may be adhered or secured to an object either directly or by forming a loop with the tag 27.

In a wristband region 22, a continuous or discontinuous die cut 42 through both the media layer 30 and the liner layer 28 defines an outline of the wristband 23. The cut 42 is configured to enable the wristband 23 to remain
reliably attached to form 20 during printing and handling but to be easily removed after printing. Over most of the area of the wristband portion 22, the adhesive layer 36 is in direct contact with the liner layer 28 such that it is effectively permanently bonded to the media layer 30 over most of the area of the wristband 23.

The wristband 23 preferably includes a closure portion 44 at one end of the wristband. The closure portion is defined by a continuous cut 46 through only the liner layer 28. The closure portion 44 is a U-shaped feature defining a void in the liner layer 28 that approximates the shape of the strap 48 on the opposite end of the wristband 23. Another release layer 50 (similar to that for tags described above) underlies this closure portion 44 such that the corresponding portion of the liner layer 28 can be separated from the adhesive layer 36. The release layer 50 is preferably silicone or any other commonly used material. Once the portion of the liner layer 28 corresponding to the closure portion 44 is removed, the strap may be inserted therein to effect an adhesive closure of the wristband 23.

The media layer 30 is preferably made from a synthetic material such as synthetic paper or polymer. In one embodiment, the media layer 30 for the label group 24 and/or tag region 25 may be the same as the media layer 30 for the wristband portion 22. In another embodiment, the media layer 30 for the labels 26 and/or tags 27 may be different from the media layer 30 for the wristband portion 22. The media layer 30 within the label group 24 and/or tag region 25 may be synthetic paper, polymer, cellulose paper, or an overcoated
paper. A non-synthetic media layer 30 used in these regions 24, 25 will decrease the overall manufacturing cost of the form 20.

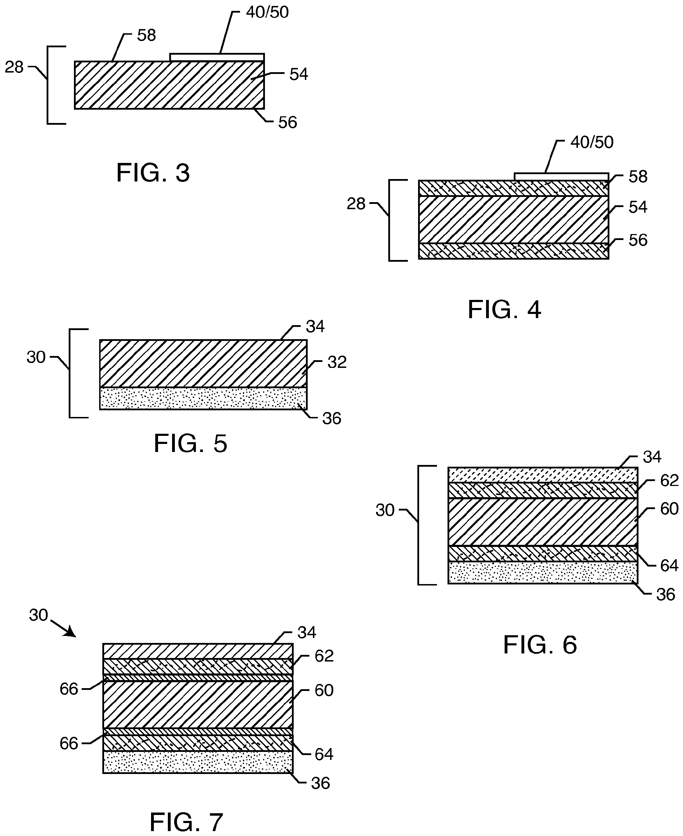

In a particularly preferred embodiment, the liner layer 28 includes a polyethylene terephthalate (PET) core layer 54 that is treated on both sides. As depicted in FIG. 3, a lower surface of the PET core layer 54 receives a slip coating 56 that optimizes the liner layer 28 for feeding through a printer. The slip coating 56 allows the pick roller in printers to advance one form 20 at a time out of a plurality of forms, i.e., singulation, and improves handle-ability of the form 20. The upper surface of the PET core layer 54 receives a surface treatment or skin layer 58 that promotes adhesion of printing inks and handling of printed information. An example of a slip coating 56 is a very thin layer of polytetrafluoroethylene (PTFE). Examples of a surface treatment 58 include a mineral filled urethane layer, as well as water based co-polymers of acrylic, polyester, polyurethane, polyvinyl acetate and polyvinylidene chloride. The adhesive layer 36, release layer 40 and/or release layer 50 are adhered directly to the surface treatment or skin layer 58.

In another preferred embodiment as illustrated in FIG. 4, the liner layer 28 may include a core or base layer 54 and upper and lower skin layers 58, 56. The core or base layer 54 may have mineral fillers such as mica dispersed throughout. The core 54 and skin layers 58, 56 are polymeric films that may be formed by coextrusion to form a multilayer film or liner layer 28. Such coextruded multilayer films are known in the industry wherein each layer is selected for particular functions such as barrier protection. The core layer 54 provides the bulk
of the chemical resistance and mechanical properties of the liner layer 28. The upper skin layer 58 provides a surface optimized to receive a release layer 40, 50, thus preventing the release layer 40, 50 from being absorbed by the core layer 54. The lower skin layer 56, while providing barrier protection, must also provide a function similar to the slip coating 56 described above.

The core layer 54 is preferably made from a polyester such as PET. However, the core layer 54 may also be made from polyolefins such as polyethylene (PE), high density polyethylene (HDPE), low density polyethylene (LDPE), or vinyl. The core layer 54 may also include fillers such as mica described above, or silica, paper pulp, ground polymer, wollastonite, glass fibers, talc, graphite platelets, graphite fibers, boron fibers, sapphire fibers, steel fibers, or polymeric or polyester fibers such as KEVLAR® (a para-aramid synthetic fiber). The fill may be about 50% by weight of the core layer 54. Typical thickness of the core layer 54 would be in the range of 50-125 microns or 2-5 mil.

The upper skin layer 58 is preferably formed from polypropylene or another material having good holdout properties of plastics for preventing the release layer 40, 50 from being absorbed into the core layer 54. The thickness of the skin layers 58, 56 would preferably be in the range of about 2-10 microns. An alternative or addition to the skin layers 58, 56 would be a surface treatment designed to achieve similar results.

As illustrated in FIG. 5, in a preferred embodiment the media layer 30 preferably includes a polymer core 32 coated with the adhesive layer 36 on one surface and an image receiving layer 34 optimized for printing on the opposing
surface. An image receiving layer 34 preferably comprises a urethane, polyethylene, polyethylene terephthalate (PET), vinyl, polyolefin, low-density polyethylene (LDPE), or high-density polyethylene (HDPE). Regardless of which polymer is used to create the image receiving layer 34, each would include a filler such as those described above for the core layer 54. The filler improves the printability of the layer.

In an alternate embodiment for the media layer 30, as shown in FIG. 6, a core layer 60 is sandwiched between an upper skin layer 62 and a lower skin layer 64. The adhesive layer 36 underlies the lower skin layer 64. The image receiving layer 34 overlies the upper skin layer 62.

The skin layers 62, 64 are preferably made from polyethylene vinyl acetate or plasticized polyvinylchloride (PVC). Alternatively, as discussed above, the skin layers 62, 64 may comprise a surface treatment. The lower skin layer 64 should be optimized for anchoring the adhesive layer 36. The image receiving layer 34 optimizes the upper surface of the media layer 30 for printing with a laser or ink-jet printer. In addition to the materials described above, the image receiving layer 34 may be a thin UV-cured urethane coating that contains a mineral filler. Laser printed images bond to an image receiving layer 60 having this type of construction very effectively. A media layer 30 so constructed provides a very durable multilayer structure.

As illustrated in FIG. 7, the media layer 30 may also include tie layers 66 between the core layer 60 and the upper and lower skin layers 62, 64. The tie layers 66 enhance the adhesion of the skin layers 62, 64 to the core layer 60 and are extremely thin, in the range of 0-5 microns. Corona discharge or other surface treatments such as plasma ashing can also be used to enhance the adhesion of the various layers or of printed material.

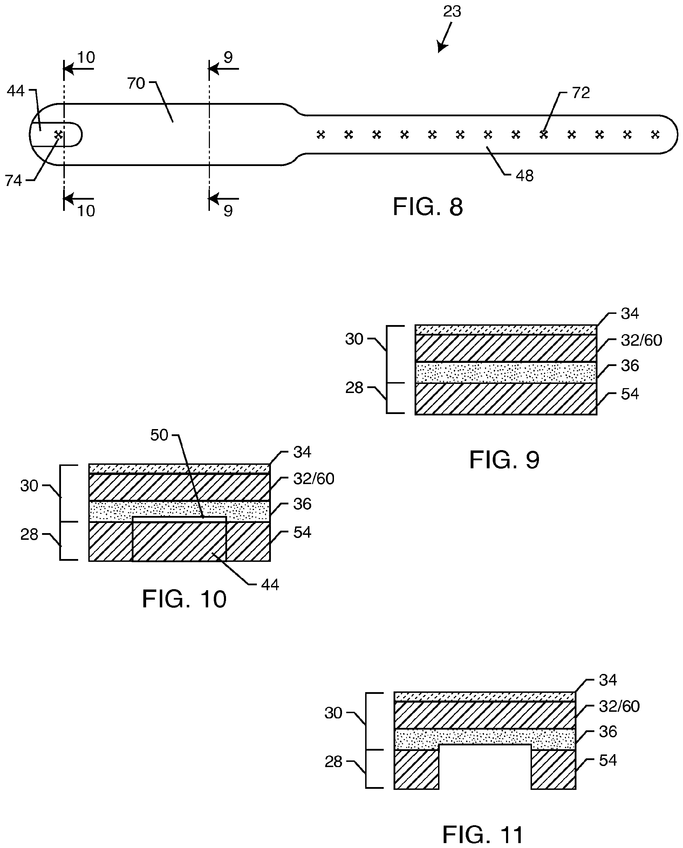

FIG. 8 illustrates a configuration of a preferred embodiment of the wristband 23. The wristband 23 includes a strap portion 48, a printable area 70 and a closure portion 44 as described above. The strap portion 48 includes a plurality of apertures 72 and the closure portion 44 includes a single aperture 74. One of the apertures 72 on the strap portion 48 may be aligned with the single aperture 74 on the closure portion 44. Alternatively, the closure portion 44 may be removed from the liner layer 28 as described above exposing the adhesive layer 36 such that the strap portion 48 may be adhered thereto.

FIGURE 9 illustrates a cross-section of wristband 23 along line 9-9 of FIG. 8. From this image, the liner layer 28 and media layer 30 are shown. The liner layer 28 is illustrated as having core layer 54. While the slip coating 56 and surface treatment 58 are not shown in this figure, they may be included as needed. The media layer 30 is shown as having core layer 32, adhesive layer 36 and image receiving layer 34.

According to FIGS. 8 and 9, media layer 30 is permanently bonded to liner layer 28 over most of the area of wristband 23. Referring back to FIG. 1, the wristband 23 is removed from form 20 by separating the wristband 23 from wristband portion 22 along outline or cut line 42. As the wristband is removed, the liner layer 28 remains bonded to media layer 30. Because liner layer 28 and media layer 30 are both polymeric layers, and because they are permanently bonded together over most of the area inside outline 44, the wristband 23 has a high durability to physical use, water, hand sanitizers, alcohols, and other environmental factors encountered by a patient or patron during sustained use of wristband 23.

FIGURE 10 illustrates a cross-section of the wristband 23 through line 10-10 in FIG. 8. In addition to showing the same layers shown in FIG. 9, FIG. 10 illustrates the construction of the closure portion 44 with the addition of the release layer 50. FIG. 10 shows the wristband prior to removal of the closure portion 44. Therefore closure portion 44 is configured to be removed by virtue of release layer 50 that allows adhesive 36 (and hence media layer 30) to be easily peeled and separated from liner 28 over the area of closure portion 44. Closure portion 44 is also easily removed because it is partially bounded by a die cut 46. In the illustrated embodiment die cut 46 is a U-shaped die cut.

FIGURE 11 illustrates the same cross-section as FIG. 10 after the removal of the closure portion 44. One can see that removal of the closure portion 44 removes a corresponding portion 44 of the liner layer 28, thereby exposing the adhesive layer 36 from beneath so that the strap portion 48 may be inserted therein and adhered thereto.

FIGURE 12 illustrates a wristband 23 having a configuration similar to that of FIG. 8. However, the layered construction of the wristband 23 shown in FIG. 12 includes additional layers as shown in FIGS. 13-15. FIG. 13 shows a cross-section of the wristband 23 in FIG. 12 along line 13-13. This cross-section is similar to that cross-section shown in FIG. 9. However, the cross-section in FIG. 13 illustrates the slip coating 56 and skin layer 58 of the liner layer 28 described above.

FIG. 13 also illustrates the upper skin layer 62, lower skin layer 64 and image receiving layer 34 of the media layer 30, also described above. Again, the adhesive layer 36 bonds the media layer 30 to the liner layer 28.

FIGURES 14 and 15 illustrate a cross-section of the wristband 23 from FIG. 12 along line 14-14. FIG. 14 illustrates the cross-section prior to removal of the closure portion 44 whereas FIG. 1 5 illustrates the cross-section after removal of the closure portion 44. Again, these figures are similar to the cross-section shown in FIGS. 10 and 11, except they illustrate the addition of the slip coating 56 and skin layer 48 in the liner layer 28 as well as the upper skin layer 62, lower skin layer 64 and image receiving layer 34 in the media layer 30. Except for the addition of these layers, the closure portion 44 functions as described above in FIGS. 10 and 11.

FIGURE 16 illustrates an alternate embodiment of the sheet 20 of the present invention. In this embodiment, the wristband region 22 and label region 24 are positioned on opposite edges of the sheet 20. The tab region 25 is positioned between the wristband region 22 and the label region 24. The construction of the liner layer 28 and media layer 30 in the sheet 20 and the various regions 22, 24, 25 are as described above. In this alternate embodiment, the media layer 30 for the wristband region 22 and the tag region 25 is generally of similar polymeric construction, whereas the media layer 30 in the label region 24 may more closely approximate synthetic paper or cellulose construction.

Although several embodiments have been described in detail for purposes of illustration, various modifications may be made without departing from the scope of the invention.

## Claims

1. A printable identification medium, comprising: a
liner layer (28); and
a printable media layer (30) disposed adjacent to the liner layer (28), the printable media layer comprising:
a core layer (32);
an image receiving layer (34) on a first side of the core layer; and
an adhesive layer (36) on an opposite second side of the core layer for bonding the printable media layer to the liner layer;
**characterized in that**
the liner layer and the printable media layer together include a wristband portion (22) and a label portion (24), the wristband portion including a wristband (23) defined by a cut (42) passing through the liner layer and the printable media layer such that the liner layer within the cut defining the wristband remains bonded to the printable media layer when the wristband is removed from the wristband portion, the label portion including labels (26) defined by cuts (38) extending through the printable media layer such that the
printable media layer may be separately removed from the liner layer within the labels and **in that** the wristband portion is comprised of a synthetic paper polymer material having fluid resistant characteristics that is capable of being printed upon by an inkjet or laser printer.

2. The printable identification medium of claim **1,** further comprising a release layer (40) between a portion of the adhesive layer (36) and the liner layer (28).

3. The printable identification medium of claims **1** or 2, wherein the identification medium further comprises a tag portion (25).

4. The printable identification medium of claim 3, wherein the tag portion (25) includes a tag (27) defined by a cut (29) passing through the liner layer (28) and the printable media layer (30).

5. The printable identification medium of claim 4, wherein the cut (29) defining the tag (27) is a discontinuous cut.

6. The printable identification medium of any of the preceding claims, wherein the cuts (38, 42) defining the wristband and labels are discontinuous cuts.

7. The printable identification medium of any of the preceding claims, wherein the cuts (38) defining the labels do not extend through the liner layer (28).

8. The printable identification medium of any of the preceding claims, further including an interior die cut (46) within the defined wristband (23) for defining a wristband attachment portion, wherein the interior die cut passes through the liner layer but not through the printable media layer.

9. The printable identification medium of claim 4, further including an interior die cut (29) within the defined tag (27) for defining a tag attachment portion, wherein the interior die cut passes through the liner layer but not through the printable media layer.

10. The printable identification medium of any of the preceding claims, wherein the core layer (32) comprises cellulose paper or synthetic paper in the label region (24).

11. The printable identification medium of any of the preceding claims, wherein the core layer (32) comprises a polymer in the wristband region (22) or the tag region (25).

12. The printable identification medium of any of the preceding claims, wherein the image receiving layer (34) comprises urethane, polyethylene, polyethylene terephthalate, vinyl, polyolefin, low-density polyethylene, or high density polyethylene, each having a filler.

## Patentansprüche

1. Ein bedruckbares Identifizierungsmedium, umfassend:
eine Trägerschicht (28); und
eine bedruckbare Medienschicht (30), die benachbart zu der Trägerschicht (28) angeordnet ist, wobei die bedruckbare Medienschicht Folgendes umfasst:
eine Kernschicht (32);
eine Bildempfangsschicht (34) auf einer ersten Seite der Kernschicht; und
eine Klebeschicht (36) auf einer gegenüberliegenden zweiten Seite der Kernschicht zum Verbinden der bedruckbaren Medienschicht mit der Trägerschicht;
**dadurch gekennzeichnet, dass**
die Trägerschicht und die bedruckbare Medienschicht zusammen einen Armbandbereich (22) und einen Etikettenbereich (24) einschließen, wobei der Armbandbereich ein Armband (23) einschließt, das definiert wird durch einen Schnitt (42), der so durch die Trägerschicht und die bedruckbare Medienschicht schneidet, daß die Trägerschicht in dem das Armband definierenden Schnitt mit der bedruckbaren Medienschicht verbunden bleibt, wenn das Armband von dem Armbandbereich entfernt wird, wobei der Etikettenbereich Etiketten (26) einschließt, die durch Schnitte (38) definiert werden, die so durch die bedruckbare Medienschicht reichen, dass die bedruckbare Medienschicht separat von der Trägerschicht in den Etiketten entfernt werden kann, und dass der Armbandbereich aus einem synthetischen Papierpolymermaterial mit flüssigkeitsresistenten Eigenschaften gebildet wird, das von einem Tintenstrahl- oder Laserdrucker bedruckt werden kann.

2. Bedruckbares Identifikationsmedium gemäß Anspruch 1, ferner umfassend eine Trennschicht (40) zwischen einem Bereich der Klebeschicht (36) und der Trägerschicht (28).

3. Bedruckbares Identifizierungsmedium gemäß einem der Ansprüche 1 oder 2, wobei das Identifizierungsmedium ferner einen Markierungsbereich (25) umfasst.

4. Bedruckbares Identifikationsmedium gemäß Anspruch 3, wobei der Markierungsbereich (25) eine Markierung (27) einschließt, die durch einen Schnitt (29) definiert wird, der durch die Trägerschicht (28) und die bedruckbare Medienschicht (30) verläuft.

5. Bedruckbares Identifikationsmedium gemäß Anspruch 4, wobei der die Markierung (27) definierende Schnitt (29) ein unterbrochener Schnitt ist.

6. Bedruckbares Identifizierungsmedium gemäß einem der vorhergehenden Ansprüche, wobei die das Armband und die Etiketten definierenden Schnitte (38, 42) unterbrochene Schnitte sind.

7. Bedruckbares Identifizierungsmedium gemäß einem der vorhergehenden Ansprüche, wobei die die Etiketten definierenden Schnitte (38) sich nicht durch die Trägerschicht (28) hindurch erstrecken.

8. Bedruckbares Identifizierungsmedium gemäß einem der vorhergehenden Ansprüche, des Weiteren einschließend einen inneren Stanzschnitt (46) innerhalb des definierten Armbands (23) zum Definieren eines Armbandbefestigungsbereichs, wobei der innere Stanzschnitt durch die Trägerschicht, aber nicht durch die bedruckbare Medienschicht verläuft.

9. Bedruckbares Identifizierungsmedium gemäß Anspruch 4, des Weiteren einschließend einen inneren Stanzschnitt (29) innerhalb der definierten Markierung (27) zum Definieren eines Markierungsbefestigungsbereichs, wobei der innere Stanzschnitt durch die Trägerschicht, aber nicht durch die bedruckbare Medienschicht verläuft.

10. Bedruckbares Identifikationsmedium gemäß einem der vorhergehenden Ansprüche, wobei die Kernschicht (32) im Etikettenbereich (24) Zellulosepapier oder synthetisches Papier umfasst.

11. Bedruckbares Identifikationsmedium gemäß einem der vorhergehenden Ansprüche, wobei die Kernschicht (32) ein Polymer im Armbandbereich (22) oder im Markierungsbereich (25) umfasst.

12. Bedruckbares Identifikationsmedium gemäß einem der vorhergehenden Ansprüche, wobei die Bildempfangsschicht (34) Urethan, Polyethylen, Polyethylenterephthalat, Vinyl, Polyolefin, Polyethylen niedriger Dichte oder Polyethylen hoher Dichte umfasst, die jeweils einen Füllstoff aufweisen.

## Revendications

1. Support d'identification imprimable comprenant :
une couche de revêtement (28) ; et
une couche de support imprimable (30) disposée de façon adjacente à la couche de revêtement (28), la couche de support imprimable comprenant :
une couche centrale (32) ;
une couche de réception d'image (34) sur un premier côté de la couche centrale ; et
une couche adhésive (36) sur un deuxième côté opposé de la couche centrale pour coller la couche de support imprimable à la couche de revêtement ;
**caractérisé en ce que**
la couche de revêtement et la couche de support imprimable comprennent ensemble une partie de bracelet (22) et une partie à étiquettes (24), la partie de bracelet comprenant un bracelet (23) défini par une découpe (42) traversant la couche de revêtement et la couche de support imprimable de manière à ce que la couche de revêtement à l'intérieur de la découpe définissant le bracelet reste collée à la couche de support imprimable lorsque le bracelet est retiré de la partie de bracelet, la partie à étiquettes comprenant des étiquettes (26) définies par des découpes (38) traversant la couche de support imprimable de manière à ce que la couche de support imprimable puisse être retirée séparément de la couche de revêtement à l'intérieur des étiquettes, et **en ce que** la partie de bracelet comprend un matériau polymère à base de papier synthétique, présentant des caractéristiques de résistance aux fluides lui permettant de recevoir une impression par une imprimante à jet d'encre ou laser.

2. Support d'identification imprimable selon la revendication 1, comprenant en outre une couche de libération (40) entre une partie de la couche adhésive (36) et la couche de revêtement (28).

3. Support d'identification imprimable selon la revendication 1 ou 2, dans lequel le support d'identification comprend en outre une partie de marquage (25).

4. Support d'identification imprimable selon la revendication 3, dans lequel la partie de marquage (25) comprend un marquage (27) défini par une découpe (29) traversant la couche de revêtement (28) et la couche de support imprimable (30).

5. Support d'identification imprimable selon la revendication 4, dans lequel la découpe (29) définissant le marquage (27) est une découpe discontinue.

6. Support d'identification imprimable selon l'une quelconque des revendications précédentes, dans lequel les découpes (38, 42) définissant le bracelet et les étiquettes sont des découpes discontinues.

7. Support d'identification imprimable selon l'une quelconque des revendications précédentes, dans lequel les découpes (38) définissant les étiquettes ne s'étendent pas à travers la couche de revêtement (28).

8. Support d'identification imprimable selon l'une quelconque des revendications précédentes, comprenant en outre un découpage à l'emporte-pièce intérieur (46) à l'intérieur du bracelet (23) défini pour définir une partie de fixation de bracelet, le découpage à l'emporte-pièce intérieur traversant la couche de revêtement mais pas la couche de support imprimable.

9. Support d'identification imprimable selon la revendication 4, comprenant en outre un découpage à l'emporte-pièce intérieur (29) dans le marquage (27) défini pour définir une partie de fixation de marquage, le découpage à l'emporte-pièce intérieur traversant la couche de revêtement mais pas la couche de support imprimable.

10. Support d'identification imprimable selon l'une quelconque des revendications précédentes, dans lequel la couche centrale (32) comprend un papier cellulosique ou un papier synthétique dans la région à étiquettes (24).

11. Support d'identification imprimable selon l'une quelconque des revendications précédentes, dans lequel la couche centrale (32) comprend un polymère dans la région de bracelet (22) ou dans la région à étiquettes (25).

12. Support d'identification imprimable selon l'une quelconque des revendications précédentes, dans lequel la couche de réception d'image (34) comprend de l'uréthane, du polyéthylène, du polyéthylène téréphtalate, du vinyle, une polyoléfine, du polyéthylène à faible densité, ou du polyéthylène à haute densité, comportant respectivement un agent de charge.
